# FASCICULE DE BREVET EUROPEEN

(11) **EP 3 979 950 B1**
(45) Date de publication et mention de la délivrance du brevet: **18.01.2023**
(21) Numéro de dépôt: 20754310.9
(22) Date de dépôt: 23.04.2020
(51) Int. Cl.: A61F 2/40

(54) **PROTHÈSE D'ÉPAULE A PARTIE EPIPHYSAIRE MODULAIRE**
SCHULTERPROTHESE MIT MODULAREM EPIPHYSALTEIL
SHOULDER PROSTHESIS WITH MODULAR EPIPHYSAL PART

(30) Priorité: 05.06.2019 FR 1905951
(43) Date de publication de la demande: 13.04.2022
(73) Titulaire: One Ortho, 69230 Saint-Genis-Laval (FR); Université Savoie Mont Blanc, 73000 Chambéry (FR); Franco-Swiss Orthoconsulting, 67205 Oberhausbergen (FR)
(72) Inventeur: VACHER, Pierre, 74570 THORENS GLIERES (FR); GOYALLON, Thibault, 74000 ANNECY (FR); VITTECOQ, Eric, 74570 GROISY (FR); DODELIN, Arnaud, 76420 BIHOREL (FR); ARLETTAZ, Yvan, 18780 MONTHEY (CH); SCHENCK, Benoit, 67205 OBERHAUSBERGEN (FR); ANTONI, Maxime, 67000 STRASBOURG (FR); ALEPEE, Christophe, 69003 LYON (FR); DESPRES, Christophe, 74370 CHARVONNEX (FR)
(74) Mandataire: Cabinet Laurent & Charras
(86) Numéro de dépôt international: PCT/FR2020/050688
(87) Numéro de publication internationale: WO 2020/245514

(56) Documents cités:
- WO-A1-2014/096912
- FR-A1- 2 876 899
- FR-A1- 2 957 517

## Description

### DOMAINE TECHNIQUE

La présente invention se rapporte au secteur technique de la chirurgie orthopédique, et plus particulièrement au domaine des prothèses totales d'épaule.

L'invention concerne une prothèse d'épaule à partie épiphysaire modulaire.

### ETAT ANTERIEUR DE LA TECHNIQUE

Il est parfaitement connu pour un homme du métier, une prothèse d'épaule à partie épiphysaire modulaire.

D'une manière générale et tel qu'illustré par le document FR 2 876 899, ce type de prothèse d'épaule comprend une partie diaphysaire s'étendant selon un axe diaphysaire, surmontée de la partie épiphysaire.

La partie épiphysaire présente une face supérieure inclinée selon un plan frontal par rapport à l'axe diaphysaire, et une face centrale inférieure d'appui contre une face complémentaire supérieure de la partie diaphysaire.

D'une manière connue, l'inclinaison de la face supérieure de la partie épiphysaire varie en fonction des différentes typologies d'implants. Le plus souvent, cette inclinaison est présente un angle plus faible pour un modèle d'implant anatomique, et plus grand pour un implant inversé.

À titre d'exemple, pour une prothèse d'épaule anatomique, la face supérieure de la partie épiphysaire est inclinée de sorte qu'un axe normal à cette face soit lui-même incliné d'un angle de 135° selon le plan frontal et par rapport à l'axe diaphysaire, tandis que pour une prothèse d'épaule inversée, l'axe normal à la face supérieure de la partie épiphysaire est incliné d'un angle de 145° selon le plan frontal et par rapport à l'axe diaphysaire.

En d'autres termes, dans une prothèse d'épaule inversée, la face supérieure de la partie épiphysaire est plus proche de l'horizontal que pour un implant anatomique, ce qui permet alors d'améliorer la cinématique de la prothèse d'épaule inversée, tout en étant plus conservateur du stock osseux du patient puisqu'il est nécessaire de couper moins de parties osseuses en interne et en en bas.

Cette variabilité entre les inclinaisons des faces supérieures des parties épiphysaires multiplie alors le nombre d'implants nécessaires pour réaliser les différentes opérations chirurgicales.

Par exemple, avec des prothèses d'épaule monobloc, en considérant deux valeurs d'angle possible, à savoir par exemple 135° et 145°, pour 4 tailles épiphysaires, et pour 4 diamètres diaphysaires, il faut mettre à disposition 32 prothèses humérales d'épaule pour répondre à toutes les configurations possibles.

Pour répondre à cet inconvénient, et diminuer le nombre d'implants nécessaires, il est bien connu de concevoir des prothèses d'épaule à partie épiphysaire modulaire, tel qu'illustré par le brevet FR 2 652 498.

Selon cet état de la technique, la partie diaphysaire devient alors commune pour les différents types de prothèses, anatomique ou inversée, ce qui simplifie la fabrication des prothèses.

Cependant, si l'on considère toujours les deux valeurs d'angle, à savoir par exemple 135° et 145°, 4 tailles épiphysaires et 4 diamètres diaphysaires, il faut mettre à disposition 8 parties épiphysaires, et 4 parties diaphysaires pour répondre à toutes les configurations possibles, de sorte qu'il est tout de même nécessaire de concevoir 12 pièces.

Une autre solution existe pour réduire ce nombre de pièces, qui consiste notamment en des parties diaphysaires communes entre les prothèses anatomiques et inversées et, dans le cas de la prothèse inversée, de concevoir des inserts en polyéthylène haute densité en forme de coin qui viennent compenser l'écart d'inclinaison entre les différentes versions.

Ainsi, si l'on considère les deux valeurs d'angle possible, à savoir par exemple 135° et 145°, 4 tailles épiphysaires et 4 diamètres diaphysaires, il faut mettre à disposition tout de même 16 pièces pour répondre à toutes les configurations possibles.

Par ailleurs, cette dernière solution présente également l'inconvénient de nécessiter une coupe osseuse plus importante dans le cas des prothèses inversées, et une sollicitation de l'insert en bas beaucoup plus importante, entraînant également une sollicitation plus importante de la fixation de l'insert sur sa platine.

### EXPOSE DE L'INVENTION

L'un des buts de l'invention est donc de remédier aux inconvénients précités en proposant une prothèse humérale d'épaule à partie épiphysaire modulaire, permettant de réaliser des prothèses dites anatomiques, par exemple avec une inclinaison de la face supérieure de la partie épiphysaire de 135°, et une prothèse dite inversée, avec une inclinaison par exemple 145°, tout en réduisant au maximum le nombre de pièces nécessaires pour couvrir toutes les configurations possibles, à savoir pour fournir par exemple différentes tailles épiphysaires et différents diamètres épiphysaires.

À cet effet, il a été mis au point, et d'une manière connue, une prothèse d'épaule comprenant une partie diaphysaire s'étendant selon un axe diaphysaire, surmontée d'une partie épiphysaire. La partie épiphysaire présente une face supérieure inclinée selon un plan frontal par rapport à l'axe diaphysaire, et une face centrale inférieure d'appui contre une face complémentaire supérieure de la partie diaphysaire.

Selon l'invention, la partie épiphysaire est réversible par rotation de 180° autour d'un axe normal à la face centrale inférieure d'appui de la partie épiphysaire, pour modifier l'inclinaison de la face supérieure de la partie épiphysaire, notamment entre ses deux positions possibles.

Ainsi, avec une même partie épiphysaire, il est possible de réaliser deux prothèses d'épaule, avec des angles différents d'inclinaison de la face supérieure de la partie épiphysaire.

Ainsi, si l'on considère deux valeurs d'angle possible pour la face supérieure de la partie épiphysaire, 4 tailles épiphysaires et 4 diamètres diaphysaires, il faut donc mettre à disposition 4 pièces pour les parties épiphysaires et 4 pièces pour les parties diaphysaires, pour répondre à toutes les configurations possibles, soit un total de 8 pièces.

Le nombre de pièces nécessaires est donc réduit, ce qui réduit par conséquent le coût de fabrication et le coût du stock nécessaire pour réaliser une telle intervention.

Selon une forme de réalisation particulière, la partie épiphysaire est apte à adopter une première position dans laquelle un axe normal à sa face supérieure est incliné d'un angle de 135° selon le plan frontal par rapport à l'axe diaphysaire, une deuxième position, pivotée de 180° autour de l'axe normal à sa face centrale inférieure d'appui, dans laquelle l'axe normal à sa face supérieure est incliné d'un angle de 145° selon le plan frontal par rapport à l'axe diaphysaire.

Selon une forme de réalisation particulière, la face centrale inférieure de la partie épiphysaire et la face complémentaire supérieure de la partie diaphysaire sont inclinées d'un angle α selon le plan frontal par rapport à la face supérieure, de sorte à permettre de modifier l'inclinaison entre la face supérieure de de la partie épiphysaire et l'axe diaphysaire d'un angle égal à +/- 2α.

Par exemple, si l'angle α est égal à 5°, et que l'axe normal de la face inférieure de la partie épiphysaire est incliné d'un angle de 140° selon le plan frontal et par rapport à l'axe diaphysaire, cela permet à l'axe normal de la face supérieure de la partie épiphysaire d'être, dans une première position, incliné d'un angle de 135° et, dans une deuxième position, incliné d'un angle de 145°.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées dans lesquelles :
[Fig. 1] la figure 1 est une représentation schématique illustrant, dans un plan frontal, une prothèse d'épaule selon l'invention, les parties épiphysaire et diaphysaire étant représentées avant assemblage et pour former une prothèse anatomique ;
[Fig. 2] la figure 2 est une représentation schématique similaire à celle de la figure 1, illustrant les parties épiphysaire et diaphysaire assemblées ;
[Fig. 3] la figure 3 est une représentation schématique similaire à celle de la figure 1, avant assemblage, pour former une prothèse d'épaule inversée ;
[Fig. 4] la figure 4 est une représentation schématique similaire à celle de la figure 3, illustrant les parties épiphysaire et diaphysaire assemblées.

### EXPOSE DETAILLE DE L'INVENTION

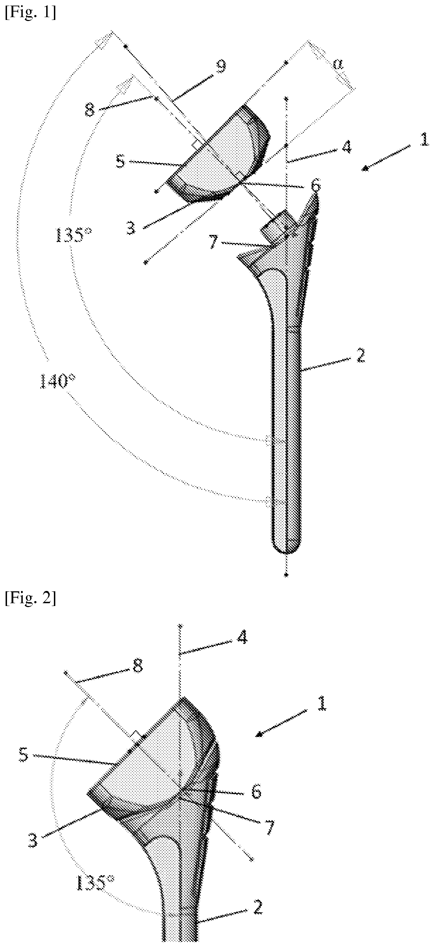

En référence aux figures 1 à 4, l'invention concerne une prothèse d'épaule modulaire (1), c'est-à-dire qu'elle comprend une partie diaphysaire (2), et une partie épiphysaire (3), distinctes et pouvant être inter-changées.

D'une manière connue, la partie diaphysaire (2) s'étend selon un axe diaphysaire (4), et est surmontée par la partie épiphysaire (3). L'assemblage entre les parties diaphysaire et épiphysaire est bien connu de l'homme du métier, de sorte qu'il ne sera pas décrit en détail.

Toujours d'une manière connue, la partie épiphysaire (3) présente une face supérieure (5) inclinée selon un plan frontal par rapport à l'axe diaphysaire (4), et une face centrale inférieure (6) d'appui contre une face complémentaire supérieure (7) de la partie diaphysaire (2).

L'inclinaison de la face supérieure (5) de la partie épiphysaire (3) dépend du type de prothèse d'épaule.

Par exemple, pour une prothèse d'épaule du type anatomique, la face supérieure (5) de la partie épiphysaire (3) doit être plus inclinée que pour une prothèse du type inversé dans laquelle la face supérieure (5) de la partie épiphysaire (3) se rapproche de l'horizontale.

En pratique, on définit l'angle d'inclinaison de la face supérieure (5) de la partie épiphysaire (3) par l'angle que forme un axe normal (8) à ladite face supérieure (5) par rapport à l'axe diaphysaire (4).

La prothèse d'épaule (1) selon l'invention permet, avec une seule et même partie épiphysaire (3), de concevoir deux prothèses d'épaule (1) dont les faces supérieures (5) des parties épiphysaires (3) présentent des angles d'inclinaison différents.

À cet effet, et selon l'invention, la partie épiphysaire (3) est réversible par rotation de 180° autour d'un axe normal (9) à la face centrale inférieure (6) d'appui de la partie épiphysaire (3) pour modifier l'inclinaison de la face supérieure (5) de la partie épiphysaire (3).

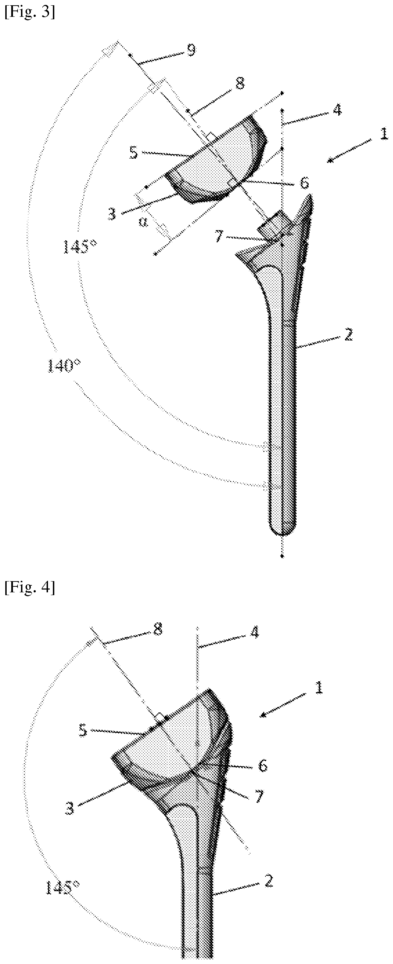

En d'autres termes, la partie épiphysaire (3) est apte à adopter une première position (voir figures 1 et 2) dans laquelle l'axe normal (8) à sa face supérieure (5) est incliné d'un premier angle, par exemple 135°, selon le plan frontal par rapport à l'axe diaphysaire (4), et une deuxième position (voir figures 3 et 4) pivotée de 180° autour de l'axe normal (9) à sa face centrale inférieure (6) d'appui, dans laquelle l'axe normal (8) à sa face supérieure (5) est incliné d'un deuxième angle, par exemple 145°, selon le plan frontal par rapport à l'axe diaphysaire (4).

De cette manière, par simple rotation de la partie épiphysaire (3), il est possible de réaliser deux types de prothèses d'épaule (1) différentes, par exemple une prothèse (1) de type anatomique, voir figures 1 et 2, et une prothèse (1) de type inversé, voir figures 3 et 4.

Pour ce faire, et selon une forme de réalisation particulière, la face centrale inférieure (6) de la partie épiphysaire (3) et la face complémentaire supérieure (7) de la partie diaphysaire (2) sont inclinées d'un angle α selon le plan frontal par rapport à la face supérieure (5), de sorte à permettre, lors du pivotement de la partie épiphysaire (3) de 180°, de faire varier l'inclinaison entre la face supérieure (5) de la partie épiphysaire (3) et l'axe diaphysaire (4) d'un angle égal à +/- 2α.

Par exemple, en référence aux figures annexées, la face centrale inférieure (6) de la partie épiphysaire (3) et la face complémentaire supérieure (7) de la partie diaphysaire (2) sont inclinées d'un angle de 5° selon le plan frontal et par rapport à la face supérieure (5).

Ainsi, dans l'exemple des figures, l'axe normal (9) à la face centrale inférieure (6) de la partie épiphysaire (3) est incliné selon le plan frontal par rapport à l'axe diaphysaire (4) d'un angle de 140°. De cette manière, la face supérieure (5) de la partie épiphysaire (3) est apte à adopter une première position, voir figures 1 et 2, dans laquelle l'axe normal (8) à ladite face supérieure (5) est incliné selon le plan frontal et par rapport à l'axe diaphysaire (4) d'un angle de 135°, et une deuxième position, voir figures 3 et 4, dans laquelle l'axe normal (8) à la face supérieure (5) de la partie épiphysaire (3) est incliné selon le plan frontal et par rapport à l'axe diaphysaire (4) d'un angle de 145°.

De ce qui précède, si l'on considère deux valeurs d'angle possible, tel que précité, à savoir 135° et 145°, 4 tailles épiphysaires et 4 diamètres diaphysaires, il est alors nécessaire de mettre à disposition 4 pièces pour les parties épiphysaires (3) et 4 pièces pour les parties diaphysaires (2) pour répondre à toutes les configurations d'implantation possibles, soit un total de 8 pièces.

De ce qui précède, la présente invention permet de diminuer le nombre de pièces nécessaires pour la conception des différentes prothèses d'épaule (1).

Bien entendu, les valeurs des angles d'inclinaison décrites ci-avant ne sont pas limitatives. Il peut tout à fait être envisagé de changer la valeur de l'angle α et/ou l'angle d'inclinaison de la face centrale inférieure (6) de la partie épiphysaire (3) et de la face complémentaire supérieure (7) de la partie diaphysaire (2) pour fournir d'autres types de prothèses (1) présentant d'autre inclinaison, avec par exemple des couples de valeurs d'angle de 130°/140° ou 140°/150°.

## Revendications

1. Prothèse d'épaule (1) comprenant une partie diaphysaire (2) s'étendant selon un axe diaphysaire (4), surmontée d'une partie épiphysaire (3), la partie épiphysaire (3) présente une face supérieure (5) inclinée selon un plan frontal par rapport à l'axe diaphysaire (4), et une face centrale inférieure (6) d'appui contre une face complémentaire supérieure (7) de la partie diaphysaire (2), ***caractérisée* en ce que** la partie épiphysaire (3) est réversible par rotation de 180° autour d'un axe normal à la face centrale inférieure (6) d'appui de la partie épiphysaire (3) pour modifier l'inclinaison de la face supérieure (5) de la partie épiphysaire (3).

2. Prothèse d'épaule selon la revendication 1, ***caractérisée* en ce que** la partie épiphysaire (3) est apte à adopter une première position dans laquelle un axe normal à sa face supérieure (5) est incliné d'un angle de 135° selon le plan frontal par rapport à l'axe diaphysaire (4), et une deuxième position pivotée de 180° autour de l'axe normal à sa face centrale inférieure (6) d'appui, dans laquelle l'axe normal à sa face supérieure (5) est incliné d'un angle de 145° selon le plan frontal par rapport à l'axe diaphysaire (4).

3. Prothèse d'épaule selon l'une des revendications précédentes, ***caractérisée* en ce que** la face centrale inférieure (6) de la partie épiphysaire (3) et la face complémentaire supérieure (7) de la partie diaphysaire (2) sont inclinées d'un angle α selon le plan frontal par rapport à la face supérieure (5), de sorte à permettre de modifier l'inclinaison entre la face supérieure (5) de la partie épiphysaire (3) et l'axe diaphysaire (4) d'un angle égal à +/- 2α.

4. Prothèse d'épaule selon la revendication 3, ***caractérisée* en ce que** l'angle α est égal à 5°.

## Patentansprüche

1. Schulterprothese (1) mit einem diaphysären Teil (2), das entlang einer diaphysären Achse (4) verläuft, überragt von einem epiphysären Teil (3), der epiphysäre Teil (3) weist dabei eine obere Fläche (5) auf, die bezogen auf die diaphysäre (4) Achse entsprechend einer frontalen Ebene geneigt ist. sowie eine untere zentrale Auflagefläche (6) gegen eine obere komplementäre Fläche (7) des diaphysären Teils (2), ***dadurch gekennzeichnet, dass*** der epiphysäre Teil (3) durch Rotation um 180° um eine normale Achse zur unteren zentralen Auflagefläche (6) des epiphysären Teils (3) reversibel ist, um die Neigung der oberen Fläche (5) des epiphysären Teils (3) zu ändern.

2. Schulterprothese nach Anspruch 1, ***dadurch gekennzeichnet, dass*** der epiphysäre Teil (3) in der Lage ist, eine erste Position einzunehmen, in der eine normale Achse auf seiner oberen Fläche (5) um einen Winkel von 135° geneigt ist, gemäß der frontal Ebene bezogen auf die diaphysäre Achse (4), und eine zweite um 180° geneigte Position um die normale Achse auf der zentralen unteren Auflagefläche (6) bei dem die normale Achse auf der oberen Fläche (5) um einen Winkel von 145° geneigt ist, entsprechend der frontalen Ebene bezogen auf die diaphysäre Achse (4).

3. Schulterprothese nach einem der vorangehenden Ansprüche, ***dadurch gekennzeichnet, dass*** die untere zentrale Fläche (6) des epiphysären Teils (3) und die komplementäre obere Fläche (7) des diaphysären Teils (2) um einen Winkel α gemäß der frontalen Ebene bezogen auf die obere Fläche (5) geneigt sind, so dass die Neigung zwischen der oberen Fläche (5) des epiphysären Teils (3) und der diaphysäre Achse (4) um einen Winkel gleich +/- 2α geändert werden kann.

4. Schulterprothese nach Anspruch 3, ***dadurch gekennzeichnet, dass*** der Winkel gleich 5° ist.

## Claims

1. Shoulder prosthesis (1) comprising a diaphyseal part (2) extending along a diaphyseal axis (4), surmounted by an epiphyseal part (3), the epiphyseal part (3) having an upper surface (5) inclined along a front plane with respect to the diaphyseal axis (4), and a lower central face (6) bearing against an upper complementary surface (7) of the diaphyseal part (2), ***characterised* in that** the epiphyseal part (3) is reversible by rotation through 180° about an axis normal to the lower central face (6) of the epiphyseal part (3) to modify the inclination of the upper surface (5) of the epiphyseal part (3).

2. Shoulder prosthesis according to claim 1, ***characterised* in that** the epiphyseal part (3) is adapted to adopt a first position in which an axis normal to its upper face (5) is inclined by an angle of 135° along the front plane with respect to the diaphyseal axis (4), and a second position pivoted by 180° about the axis normal to its lower central face (6), in which the axis normal to its upper face (5) is inclined by an angle of 145° along the front plane with respect to the diaphyseal axis (4).

3. Shoulder prosthesis according to one of the preceding claims, ***characterised* in that** the lower central face (6) of the epiphyseal part (3) and the complementary upper face (7) of the diaphyseal part (2) are inclined by an angle α along the front plane with respect to the upper face (5), so as to make it possible to modify the inclination between the upper face (5) of the epiphyseal part (3) and the diaphyseal axis (4) by an angle equal to +/-2a.

4. Shoulder prosthesis according to claim 3, ***characterised* in that** the angle α is equal to 5°.
